Europäisches Patentamt

European Patent Office

Office européen des brevets

(11) Publication number: **0 443 063 A1**

# EUROPEAN PATENT APPLICATION

(21) Application number: 90103382.9

(22) Date of filing: 22.02.90

(51) Int. Cl.⁵: **C12N 15/55**, C12N 1/21, C12N 9/20, C12P 7/62, C12P 17/02, C11D 3/386, //C12R1/38

The microorganism(s) has (have) been deposited with American Type Culture Collection under numbers ATCC 68181 - 68160.

The title of the invention has been amended (Guidelines for Examination in the EPO, A-III, 7.3).

(43) Date of publication of application:
28.08.91 Bulletin 91/35

(84) Designated Contracting States:
AT BE CH DE ES FR GB GR IT LI LU NL SE

(71) Applicant: **HENKEL RESEARCH CORPORATION**
**2330 Circadian Way**
**Santa Rosa California 95407(US)**

(72) Inventor: **Hom, Sherman Siu Ming**
**1237 St. Francis Road**
**Santa Rosa, California 95409(US)**
Inventor: **Mielenz, Jonathan Richard**
**1078 Ferguson Road**
**Sebastopol, California 95472(US)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) Pseudomonas lipase gene, vectors for the expression thereof, production of the lipase by transformed microorganisms and uses of this enzyme.

(57) Nucleic acid constructs are provided comprising a lipase gene, which constructs are introduced into appropriate bacterial hosts, particularly as stable episomal elements, for efficient expression and secretion of lipase. Particularly, a pseudomonad lipase gene is employed.

EP 0 443 063 A1

## INTRODUCTION

### Technical Field

The subject field concerns the preparation of lipase in Gram-negative hosts and the use of the hosts and lipase.

### Background

Microorganisms produce a wide variety of products which are of commercial interest. In many instances, the natural production of the products is not useful, because of low yields, difficulties in isolating and purifying the product, high costs of production, and the like. In some instances, a particular host may not be amenable to transformation, efficient production, or scale-up to commercially useful levels of production. In other instances, yields may be transient or fluctuating; particularly where the gene expressing the protein of interest is on a plasmid, the plasmid may prove to be unstable. There is, therefore, an interest in being able to modify organisms to allow for efficient production of naturally occurring products of interest.

Recombinant DNA technology offers an opportunity to modify cells. The techniques involve utilizing sequences encoding for a peptide product, creating chimeric genes by joining a transcriptional initiation region associated with one gene to an open reading frame expressing other than the natural peptide, providing for additional copies of a particular gene, selecting hosts which may provide for improved phenotype, and the like. These processes are labor intensive and require extensive experimentation in isolating and defining the sequence, modifying the structure as appropriate, transforming into a host, and demonstrating product formation. Each of these steps involves extensive manipulation, proof that the desired manipulation has occurred and that mutations have not been introduced. Each construct carries with it uncertainties as to the effect of the novel DNA sequence on the host cell, and to what extent the host cell will be able to adapt to the novel sequence and provide for expression. Finally, there is the nature of the lipase, its characteristics and whether it can find commercial use.

### Relevant Literature ` `

A number of references are concerned with the cloning and identification of microbial lipases. See, for example, Japanese Patent Application No. 59-43899, Publication No. 60-188072, published September 25, 1985; Odera et al., J. Ferment. Technol. (1986) 64:363-371; Kujimiya et al., Biochem. Biophys. Res. Comm. (1986) 141:185-189; Haas, J.A.O.C.S. (1987) 64, Abstract No. 142: Gotz et al., Nucleic Acids Res. (1985) 13:5895-5906; and WO86/06743.

A number of references have studied the properties of lipases from a variety of microorganisms. See, for example, Lee and Iandolo, J. Bacteriology (1985) 164:288-293; (1986) 166:385-391; Seitz, J.A.O.C.S. - (1974) 51:12-16; Kosugi and Kamibayashi, J. Ferment. Technol. (1971) 49:968-980; Lu and Liska, Appl. Micro. (1969) 18:108-113; Nishio et al., Agric. Biol. Chem. (1987) 51:181-183; Ammar and McDaniel, Mikrobiol. (1984) 139:61-68; and Kokusho et al., Agric. Biol. Chem. (1982) 46:1159-1164; (1982) 46:1743-1750.

References concerned with broad host range plasmids include Ditta et al., Proc. Natl. Acad. Sci. USA, (1980) 77:7347-7351; Itoh et al., Plasmid (1984) 11:206-220; Itoh and Haas, Gene (1985) 36:27-36; Frey et al., Gene (1983) 24:299-308; Bagdasarian et al., Gene (1981) 16:237-247; and Friedman et al., Gene (1982) 18:289-296.

Patents of interest concerned with lipases include JP86/54508 (JP622/10981); JP86/54509 (JP622/10986); JP86/54510 (JP622/10987); EP86/72307 (JP622/28279); EP0238023; EP0243338.

## SUMMARY OF THE INVENTION

Expression constructs, vectors, transformed hosts and methods of using the hosts are provided for the efficient production of a specific Pseudomonas lipase enzyme having desirable characteristics. The organisms and product find use as a component in detergent and cleaning products for lipid stain removal, in the hydrolysis and production of esters, i.e. monoglycerides, resolution of ester stereoisomers, cyclization of hydroxy-substituted dicarboxylic acids to form lactones, which are precursors for synthesis of fragrance molecules, and production of peracids.

## DESCRIPTION OF THE SPECIFIC EMBODIMENTS

EP 0 443 063 A1

DNA constructs are provided comprising a Pseudomonas lipase gene. The construct may be transformed into a suitable host, particularly as a stable episomal element, for expression of the lipase. The lipase may be isolated and used in a variety of commercial applications.

The lipase gene which is employed is a Pseudomonas lipase capable of secretion and present in Pseudomonas sp. ATCC 21808. This gene or congeners thereof may be obtained by transforming a Lip⁻ host (a host that lacks lipase activity) with vectors comprising fragments from the indicated strain. Various Pseudomonas Lip⁻ strains exist, such as P. oleovorans ATCC 8062 Rifʳ strain. The presence of Lip⁺ transformants (a host possessing lipase activity) may be detected by competence to hydrolyze lipid esters, such as castor oil.

Once a fragment containing the gene has been identified, the fragment may be manipulated in a variety of ways. Particularly, where the original library or gene bank comprises fragments of 10 kbp or more, the fragments may be subjected to further restriction employing different endonucleases to provide for fragments in the range of about 2 to 10 kbp. The smaller fragment has many advantages in reducing the number of available restriction sites, ease of manipulation, avoidance of sequences which may be detrimental, and the like.

In addition, one may wish to identify the open reading frame and the 5'-untranslated region, which serves as the transcriptional and translational initiation regulatory region. The regulatory region may be modified in a variety of ways, substituting the transcriptional initiation region with a different region, joining regions which provide for inducible regulation of transcription, for example, by changes in temperature, presence or absence of metabolites, or the like. Alternatively, additional promoter regions may be added or substituted, so as to have two or more transcriptional initiation regions in tandem.

Transcriptional initiation regions which may find use in Pseudomonas include the regions for β-galactosidase, α-amylase, glycerol-6-phosphate dehydrogenase, alkaline phosphatase, protease, toluene degradation, xyl promoter, the tac promoter, etc. The regions may be endogenous to the Pseudomonas species host or exogenous to Pseudomonas such as regions functional in Pseudomonas from E. coli, Bacillus, etc.

The restriction fragments may be selected to include the complete gene, including the regulatory regions necessary for expression. Generally, the native fragment will be at least about 0.5 kbp, more usually 3 kbp, and not more than about 10 kbp, more usually not more than about 8 kbp. In some instances, it may be desirable to manipulate the sequence, by replacing the 5' untranslated region with a different regulatory region. The 5' untranslated region may be removed by restriction, resection, primer repair, in vitro mutagenesis, or the like. All or a portion of the structural gene encoding the lipase may be synthesized, particularly where one or more mutations are introduced into the lipase by modifying, inserting or deleting base pairs off the structural gene.

The cloning vector may also serve as the expression vector, where the cloning vector has a replication system functional in the host. Particularly, where the fragments are identified by expression of the lipase in a negative background, the vector may be isolated and transformed into the expression host for production of the desired lipase. Alternatively, the gene may be isolated, manipulated and inserted into a different vector for transformation and expression of the lipase gene.

The vector is characterized by having at least one replication system and may have two or more. For example, shuttle vectors may be employed where replication systems are present for a cloning host, e.g. E. coli, and an expression host, e.g. Pseudomonas spp. In addition, there are usually one or more markers for selection of host cells transformed with the vector. Markers may be biocide resistance, e.g. antibiotic resistance, imparting prototrophy to an auxotrophic host, or the like. Other functions which may be present are mobilizability, but not transmissibility, high copy number, runaway replication system, particularly temperature sensitive, polylinker(s), etc.

The vector may be introduced into the host by any convenient means, such as transfection, conjugation, transformation, electroporation, injection, fusion, transduction, or the like, all of which provide for the introduction of DNA into a cellular host and replication of such DNA.

In some instances, to enhance the number of copies of the lipase gene, it may be desirable to have a plurality of multicopy plasmids present in the same host. This is feasible where the replication systems are from different incompatibility groups, e.g. different P incompatibility groups.

While expression of the lipase may be performed in any convenient host, particularly procaryotic hosts, for the most part, it will be desirable that the subject vector be transformed into a Pseudomonas host, more particularly the Pseudomonas host from which the gene was isolated. By selecting a Pseudomonas host, which is already a relatively high producer of the lipase (a positive background selected for high production), improved results may be obtained.

The recombinant expression vector should be a multi-copy vector, providing at least 5, usually at least

3

10 copies, preferably at least about 15 copies, and may be 100 or more copies, generally not more than about 20 copies. The transformed host may produce at least 10 times the amount of lipase naturally produced by the host, preferably at least about 15 times, and more preferably at least about 20 times or more than the untransformed host. The untransformed host will generally be at a level of 5-20 U/ml under the conditions described in the Experimental section during a period of from about 24 to 96 hours.

The lipases of interest are characterized by being thermostable, being active over a broad pH range and having an optimum on the alkaline side. In addition, the lipases will generally be secreted.

Particular replication systems include plasmids derived from P-1 or P-4 incompatibility group plasmids, e.g. pKT231 (Badsarian et al., Gene (1981) 16:237-247), RP4, RK2, pVS1 (Itoh and Hass, Gene (1985) 36:27-36), pRK290, etc. Specific hosts of interest include Pseudomonas oleovorans, Pseudomonas sp. ATCC 21808, Pseudomonas putida, Pseudomonas aeruginosa, Pseudomonas fluorescens, Bacillus subtilis, and Bacillus licheniformis.

The transformants will be grown under conditions which support viability and enhance the stable production of lipase. Cell densities will generally be in the range of about $10^9$ to $10^{11}$ cells/ml. The temperature will generally be in the range of 28° to 39° C. Nutrient media which find use include PYEO (see Example 5, infra.) Other conditions normally employed are rotary shaking (250 rpm) and Erlenmeyer flasks containing baffles.

The lipase may be isolated in conventional ways by separation of the cells, concentration of the supernatant or filtrate, precipitation with ethanol, with redispersion and reprecipitation as appropriate. The enzyme may be further purified by using reverse phase chromatography and preparative isoelectric focusing.

Of particular interest is a mature secreted lipase enzyme having an amino terminus having substantially the following amino acid and DNA sequence at the N-terminus:

| Ala | Asp | Asn | Tyr | Ala | Ala | Thr | Arg | Tyr | Pro | Ile |
|-----|-----|-----|-----|-----|-----|-----|-----|-----|-----|-----|
| 5' GCC | GAC | AAC | TAC | GCG | GCG | ACG | CGT | TAT | CCG | ATC |

The putative active site amino acid and encoding DNA sequence are as follows,

| Leu | Val | Gly | His | Ser | Gln | Gly | Gly |
|-----|-----|-----|-----|-----|-----|-----|-----|
| 5' CTC | GTC | GGC | CAC | AGC | CAG | GGC | GGG |

where the underlined amino acids appear to be conserved in lipases from a plurality of sources.

The subject compositions find wide applications for use in the hydrolysis of esters, particularly the hydrolysis of fats to produce fatty acids and monoglycerides. Alternatively, the process may be reversed, employing the lipase to esterify polyols such as glycerol or sugars. In addition, the lipases may be used for enantioselective hydrolysis of esters to provide for enantiomerically enriched synthons. Thus, by employing esters comprising stereoisomeric fatty acids and/or esters, a product may be obtained which is enriched as to a specific enantiomer.

The lipase of the instant invention can be useful as a lipolytic detergent additive in both liquid and granular detergents. Detergent compositions generally contain as essential components surfactants, detergency builders, bleaching or fluorescent whitening agents and auxiliary agents. Auxiliary agents may be enzymes, such as proteolytic and lipolytic enzymes. The lipase should be stable in detergent solution in the presence of commonly used detergent proteases. Furthermore, the lipase described herein can provide improved cleaning performance and better stability, particularly on enzyme sensitive stains such as fats. Additionally, said lipase may be used in an activated system for in situ generation of peracid for bleaching.

Detergent compositions employing lipases have found exemplification in the literature. See, for example, EPA 258008, whose disclosure is incorporated herein by reference, particularly page 4, line 22 to page 5, line 24.

The following examples are offered by way of illustration and not by limitation.

EXPERIMENTAL

Example 1

Construction of a Pseudomonas sp. ATCC 21808 DNA Gene Bank

Part 1: Isolation of the genomic DNA from P. sp. ATCC 21808 (American Type Culture Collection, Rockville, MD).

Genomic DNA was isolated from Pseudomonas sp. ATCC 21808, which secretes a lipase enzyme and was originally isolated from soil by scientists at Amano Pharmaceutical Co., Ltd. (Nagoya, Japan). Its microbiological properties, i.e. morphology, cultural characteristics, and other biological properties are described in U.S. Patent No. 3,875,007; entitled "Lipid Metabolism Improving and Anti-Atheromatic Agent" in columns 7-9. P. sp. ATCC 21808 bacteria are Gram-negative non-spore forming polarly flagellated aerobic rods.

The P. sp. ATCC 21808 cells were grown overnight at 30°C in nutrient broth, which consisted of 3 grams of beef extract (Difco laboratories, Detroit, MI) and 5 grams of peptone (Difco Laboratories, Detroit, MI) per liter.

The bacteria were harvested by centrifugation at 2,950 x g for 20 min at 4°C and resuspended in a 0.05 M Tris (Sigma Chemical Co., St. Louis, MO) and 0.05 M ethylenediaminetetraacetic acid (EDTA) (Sigma Chemical Co., St. Louis, MO) at pH 8. The cells were then treated with lysozyme (1 mg/ml) (Sigma Chemical Co., St. Louis, MO) for 45 min at 4°C prior to lysis. The cells were lysed with a 1/4 volume of detergent solution, which contained 0.5% (w/v) sodium dodecyl sulfate (SDS) (Pierce Chemical Co., Rockford, IL), 0.05 M Tris pH 7.5, and 0.4 M EDTA. The cell lysis was completed by incubating the ruptured cells at 50°C for 1 hr. Moreover, the proteins in the cell lysate were hydrolyzed with proteinase K (Sigma Chemical Co., St. Louis, MO) (1 mg/ml) during the above-mentioned heat step.

The nucleic acids (DNA and RNA) were separated from the other cellular components by extracting twice with unbuffered phenol/chloroform (1:1, v/v) (International Biotechnologies Inc., New Haven, CT) and once with chloroform/isoamyl alcohol (Sigma Chemical Co., St. Louis, MO) (24:1, v/v). The nucleic acids were precipitated by adding 1/2 vol of 7.5 M ammonium acetate (International Biotechnologies Inc., New Haven, CT) and 2 vol of cold 100% ethanol (International Biotechnologies Inc., New Haven, CT). The nucleic acids were spooled onto a sterile glass Pasteur pipet. The nucleic acids were redissolved in a solution containing 0.05 M Tris pH 7.5, 1 mM EDTA, and 200 μg/ml RNase A (Sigma Chemical Co., St. Louis, MO). The RNase A hydrolyzed the contaminating RNA. The DNA was re-extracted with chloroform/isoamyl alcohol, reprecipitated as described above, spooled, and redissolved in 0.05 M Tris pH 7.5 and 1 mM EDTA. Approximately 500 μg of DNA was isolated from a 50 ml culture of P. sp. ATCC 21808 cells.

Part 2: Isolation of plasmid vector pCP13 DNA.

Plasmid pCP13 (F. Ausubel, Dept. of Molecular Biology, Massachusetts General Hospital, Boston, MA) is a broad host range cosmid vector derived from plasmid pLAFR1, which was described in Friedman et al., Gene (1982) 18:289-296. Vector pCP13 is 23 kilobases (Kb) in size, encodes for tetracycline (Tc) and kanamycin (Km) resistance, contains many unique endonuclease restriction sites, and is mobilizable (Mob⁺) but not self-transmissible (Tra⁻).

Plasmid pCP13 was isolated from cells of Escherichia coli strain HB101 by the method of Itoh et al., Plasmid (1984) 11:206-220, with some modifications. E. coli HB101 (pCP13) cells were grown overnight with aeration at 32°C on a rotary shaker (175 rpm) in Luria broth medium, which is composed of (grams per liter) 10 g of tryptone (Difco Laboratories, Detroit MI), 5 g of yeast extract (Difco Laboratories, Detroit, MI), and 10 g of sodium chloride (NaCl) (Sigma Chemical Co., St. Louis, MO). This medium was supplemented with 23 μg/ml tetracycline (Tc) (Calbiochem, La Jolla, CA). The E. coli cells were harvested by centrifugation at 5,000 x g for 10 min at 4°C. The cells were lysed with a detergent solution containing 20 cetyl ether (Brij-58) (Sigma Chemical Co., St. Louis, MO), and sodium deoxycholate (Sigma Chemical Co., St. Louis, MO). When lysis had occurred (10-20 min), a cleared lysate was obtained by centrifugation at 39,000 x g at 4°C for 90 min. After the upper phase was decanted, a 1/5 volume of PEG6000 (polyethylene glycol) (Sigma Chemical Co., St. Louis, MO), and 3% (w/v) sodium chloride was added. The lysate was incubated overnight at 4°C to allow complete precipitation of the plasmid DNA.

The precipitated plasmid DNA was collected by centrifugation at 14,000 x g for 10 min at 4°C. The DNA pellet was dissolved in 0.05 M Tris, pH 8.0, 5 mM EDTA, and 0.05 M sodium chloride, and then purified in a cesium chloride gradient using 9 g of cesium chloride (International Biotechnologies Inc., New Haven, CT) and 0.75 ml 10 mg/ml ethidium bromide (Sigma Chemical Co., St. Louis, MO). After incubating the DNA solution for 60 min at 4°C, the flocculated polyethylene glycol precipitate was separated from the solubilized plasmid DNA by centrifugation at 12,000 x g for 45 min at 4°C. The plasmid DNA was separated from chromosomal DNA by ultracentrifugation in a Beckman Ti70.1 rotor at 45,000 rpm at 18°C for 45 h. The lower band consisting of plasmid pCP13 DNA was collected with a syringe. Ethidium bromide was removed by three extractions with n-butanol (Sigma Chemical Co., St. Louis, MO) equilibrated against saturated cesium chloride in $H_2O$. The cesium chloride was removed by dialysis against three changes of 0.01 M Tris, 1 mM EDTA pH 8.0 (TE) buffer. Approximately 400 $\mu$g of pCP13 DNA was isolated from a one-liter culture.

Part 3: Isolation of 15-30 Kb size fragments of P. sp. ATCC 21808 DNA.

Total DNA from P. sp. ATCC 21808 (550 $\mu$g) and 31.25 units (U) of restriction enzyme XhoI (New England Biolabs, Beverly, MA) were mixed together in a 7.5 ml solution, which contained 0.025 M Tris-hydrochloride (Tris-HCl) at PH 7.8, 0.05 M NaCl, 0.01 M magnesium chloride ($MgCl_2$), 100 $\mu$g/ml Bovine Serum Albumin (BSA), and incubated at 37$^\circ$C for 1 h. One unit of restriction enzyme is defined as the amount of enzyme required to completely digest 1 $\mu$g of lambda DNA in 60 min at 37$^\circ$C in a total volume of 0.05 ml. Then the restriction enzyme was removed from the digestion mixture by extracting twice with unbuffered phenol/chloroform (1:1, v/v) and once with chloroform/isoamyl alcohol (24:1, v/v). The digested DNA was precipitated by adding 0.5 vol of 7.5 M ammonium acetate and 2 vol of cold 100% ethanol. After incubating for 30 min at -70$^\circ$C, the precipitated DNA was harvested by centrifugation at 13,000 x g for 30 min at 4$^\circ$C in an Eppendorf centrifuge (5415; Brinkman Instruments, Inc., Westbury, NY). After the DNA pellet was washed once with 70% cold ethanol, the DNA was redissolved in 1 ml of TE buffer pH 8.0. A small aliquot of the partially digested DNA was analysed by agarose gel electrophoresis. This analysis indicated that a majority of the DNA was greater than 10 Kb in length.

The 15-30 Kb size fragments were isolated by sucrose density centrifugation. The continuous (10-40%; w/v) sucrose gradients were constructed by filling polyallomer ultracentrifuge tubes (Beckman Instruments, Inc., Palo Alto, CA), with 11.4 ml of a 25% (w/v) sucrose (International Biotechnologies Inc., New Haven, CT) solution prepared in 0.02 M Tris pH 8, 5 mM EDTA, and 1 M NaCl and freezing the solution at -20$^\circ$C. After slowly thawing the sucrose solution at 4$^\circ$C for 4 h, the partially digested DNA solution was readjusted to 1 M NaCl, 0.02 M Tris, pH 8, and 5 mM EDTA and applied to the top of the continuous sucrose gradient. Ultracentrifugation was carried out in a Beckman SW40Ti rotor at 26,000 rpm for 24 h at 18$^\circ$C. Fractions were collected, and the DNA content was analysed by agarose gel electrophoresis. Fractions containing the 15-30 Kb size DNA were combined. Then the sucrose in the DNA solution was removed and the solution was concentrated with a Centricon-30 microconcentrator device (Amicon Corp., Danvers, MA).

Part 4: Recombining the P. sp. ATCC 21808 DNA with plasmid cloning vector pCP13.

Sixty micrograms of plasmid pCP13 DNA and 180 U of the restriction enzyme XhoI were combined in 250 $\mu$l of the same digestion buffer as described in the previous section and incubated at 37$^\circ$C for 18 h. Analysis of the DNA by agarose gel electrophoresis indicated that the digestion was complete. Proteins and other contaminating materials were removed from the digestion mixture by extracting twice with unbuffered phenol/chloroform (1:1, v/v) and once with chloroform/isoamyl alcohol (24:1, v/v). The plasmid DNA was precipitated with 0.5 vol of 7.5 M ammonium acetate and 2 vol of cold 100% ethanol. After incubating for 20 min at -70$^\circ$C, the precipitated DNA was harvested by centrifugation in an Eppendorf centrifuge at 13,000 x g for 20 min at 4$^\circ$C. After the plasmid DNA pellet was washed three times with 70% cold ethanol, the DNA was redissolved in 120 $\mu$l of TE buffer.

XhoI digested pCP13 DNA was dephosphorylated to prevent plasmid recircularization in the subsequent ligation reaction. This was accomplished in the following manner. Two 10 $\mu$g aliquots of digested pCP13 DNA and 22 U of calf intestinal alkaline phosphatase (Boehringer Mannheim Biochemicals, Indianapolis, IN) were combined in a 200 $\mu$l solution, which contained 0.05 M Tris pH 9.5 and 25 $\mu$g/ml spermidine (Sigma Chemical Co., St. Louis, MO), and incubated at 37$^\circ$C for 60 min. One unit of alkaline phosphatase is defined as the amount of enzyme that hydrolyzes 1 $\mu$mole of 4-nitrophenylphosphate in 1 min at 37$^\circ$C in a buffer, which consists of 1 M diethanolamine, 0.01 M 4-nitrophenylphosphate, 0.025 M $MgCl_2$, pH 9.8. After inactivating the phosphatase by incubating at 68$^\circ$C for 10 min, the contaminating proteins were removed by a similar round of phenol/chloroform and chloroform/isoamyl alcohol extractions as described in the previous paragraph. Moreover, the dephosphorylated pCP13 DNA was precipitated, incubated overnight at -20$^\circ$C, and washed with 70% ethanol as previously described. After drying the plasmid DNA pellet for 10 min in a Speed-Vac Concentrator (Savant Instruments Inc., Farmingdale, NY), the plasmid DNA was redissolved in TE buffer.

Then 0.5 $\mu$g of the digested and size-selected P. sp. ATCC 21808 DNA, 4.5 $\mu$g of the digested pCP13 DNA and 20 U of T4 DNA ligase (New England Biolabs, Beverly, MA) were mixed in 20 $\mu$l of a solution containing 0.025 M Tris-HCl (pH 7.8), 0.1 M $MgCl_2$, 0.04 M $\beta$-mercaptoethanol, and 4 mM adenosine 5'-triphosphate (ATP), and incubated for 16 h at 4° C. One unit of ligase is defined as the amount required to give 50% ligation of HindIII fragments of lambda DNA in 30 min at 16° C in 20 µl of a solution (which consists of 0.05 M Tris-HCl pH 7.8, 0.01 M MgCl₂, 0.02 M dithiothreitol, 1 mM ATP, 50 µg/ml BSA) and a 5' DNA termini concentration of 0.12 µM (300 µg/ml). The vector to insert ratio of 10:1 (w/w) was used to enhance the formation of long DNA concatemers, the preferred substrate for in vitro packaging of DNA molecules in lambda virus particles.

Part 5: In vitro packaging of plasmids containing P. sp. ATCC 21808 DNA into lambda particles.

In vitro packaging is an efficient means of introducing recombinant cosmids into host cells. After the Packagene extract (Promega Biotec, Madison, WI) was thawed on ice, half of the P. sp. ATCC 21808 DNA/pCP13 ligation mixture was added and the mixture was incubated at 22° C for 2 h. Then the packaging mixture was diluted with 0.5 ml of 0.1 M NaCl, 0.01 M Tris pH 7.9, and 0.01 M magnesium sulfate (MgSO₄) (Sigma Chemical Co., St. Louis, MO). The lambda particles (some containing recombinant pCP13/P. sp. ATCC 21808 DNA molecules) were stored at 4° C over chloroform.

Part 6: Transduction of plasmids containing P. sp. ATCC 21808 DNA and an antibiotic resistance marker into E. coli.

E. coli strain HB101 was grown in a flask overnight at 30° C in a rotary shaker at 175 rpm in a Tryptone broth (TB) medium which is composed of (grams per liter) 10 g of tryptone, 5 g of NaCl, 2 g maltose (Sigma Chemical Co., St. Louis, MO), and 2.46 g MgSO₄. 100 µl of resuspended HB101 bacteria (in 0.01 M MgSO₄) and 50 µl of the packaging mixture which contained lambda particles were mixed together in tubes and incubated at 37° C for 20 min. Then 1 ml of prewarmed Luria broth medium was added and the tubes were incubated at 37° C for 60 min in a rotary shaker at 150 rpm. The transduced HB101 cells were harvested by centrifugation for 5 min at room temperature at 13,000 x g in an Eppendorf centrifuge and subsequently resuspended in a small volume of Luria broth medium. The HB101 cells were grown on Luria agar plates containing 15 µg/ml Tc. This selective medium allowed the growth of only the E. coli HB101 cells which contained the plasmid pCP13 (with tetracycline resistance gene). To determine the number of cells containing recombinant DNA, the Tc resistant cells were screened for resistance to Km (Sigma Chemical Co., St. Louis, MO). Since insertion of a fragment of DNA into the unique XhoI restriction site of pCP13 inactivates the plasmid gene encoding for Km resistance, the frequency of Km sensitivity gives the number of E. coli cells containing a recombinant pCP13 plasmid in the population of cells. Each Tc resistant cell which was also Km sensitive, indicated that it had pCP13 plasmids containing P. sp. ATCC 21808 DNA. Twelve hundred HB101 recombinant strains were individually isolated.

Example 2

Isolation of Lipase-producing Strains of Pseudomonas oleovorans ATCC 8062

Part 1: Preparation of pools of E. coli HB101 recombinant strains.

The twelve hundred independently isolated Tc^rKm^s HB101 strains, which possessed a recombinant (P. sp. ATCC 21808/pCP13) DNA plasmid were spotted on the surface of a Luria agar plate supplemented with 15 µg/ml Tc (50 clones per plate). After incubating the agar plates at 37° C for 10 h, 3 ml of Luria broth containing 15 µg/ml Tc was added to each plate and the bacteria on each plate were scraped together. The resuspended cells from two plates (100 HB101 clones) were combined, thereby forming 12 pools of HB101 clones.

Part 2: Transfer of recombinant plasmids containing P. sp. ATCC 21808 DNA from E. coli to P. oleovorans by conjugation.

The donor and recipient cells were prepared for conjugation by the method of Hom et al., J. of Bacteriology (1984) 159:335-340, with some modifications.
Tubes containing 3 ml of Luria broth medium were inoculated with the E. coli (donor) pools from a Luria agar (+ 15 µg/ml Tc) stock plate and incubated overnight at 30° C in a roller drum (New Brunswick Scientific Corp., New Brunswick, NJ). These overnight cultures were used to inoculate 10 ml of the same medium to an A₄₂₀ (optical scattering at a wavelength of 420 nm) of 0.15. These cultures were incubated on a rotary shaker at 200 rpm at 37° C for 3 h.

A tube containing 3 ml of Luria broth medium containing 15 µg/ml Km was inoculated with E. coli HB101 harboring plasmid pRK2013 from a stock plate and incubated in a roller drum as described above. The overnight culture was used to inoculate 60 ml of the same medium to an A₄₂₀ of 0.1. This culture was also incubated at 200 rpm at 37° C for 3 h. Plasmid pRK2013 is a kanamycin-resistant helper plasmid which consists of the RK2 transfer genes cloned onto a ColEI replicon. (G. Ditta, Dept. of Biology, Univ. of California at San Diego, La Jolla, CA). It supplies the necessary proteins for effective mobilization (transfer) of the pCP13 recombinant plasmids and is described in detail in Ditta et al., Proc. Natl. Acad. Sci. USA - (1980) 77:7347-7351.

A flask containing 10 ml of nutrient broth containing 10 µg/ml rifampicin (Sigma Chemical Co., St. Louis, MO) was inoculated with P. oleovorans ATCC 8062 (American Type Culture Collection, Rockville, MD) Rifʳ recipient cells from a stock plate containing the same medium and incubated overnight at 30° C on a rotary shaker at 200 rpm. The overnight culture was used to inoculate 60 ml of the same medium to an A₄₂₀ of 0.3. The culture was also incubated as described above. P. oleovorans ATCC 8062 is described in detail in J. Bacteriology (1941) 41:373-386.

Both donor and recipient cells were harvested by centrifugation at 4,400 x g for 10 min at 4° C. The donor and recipient cells were washed once with Luria broth and nutrient broth respectively to remove the antibiotics. Finally, the bacteria were resuspended in 0.25 the culture volume with nutrient broth.

The HB101 clone pools, HB101 (pRK2013) helper cells, and P. oleovorans recipients were mixed together in a ratio of 5 donor and 5 helper plasmid cells per recipient cell, collected onto a Metricel membrane filter (25 mm in diameter, 0.45-µm pore size; Gelman Instrument Co., Ann Arbor, MI), and incubated on nutrient agar plates at 30° C for 2 days. The cells were then resuspended in 5 ml of nutrient broth, serially diluted, and 50 µl aliquots were spread on nutrient agar plates supplemeted with 50 µl/ml rifampicin, 25 µg/ml Tc, and 0.05% (v/v) castor oil (Sigma Chemical Co., St. Louis, MO). These plates were incubated at 30° C for 10 days. Castor oil was emulsified into the preparation, which formed a translucent agar medium. This agar medium was used to detect any P. oleovorans cells that received a lipase gene capable of expression on a recombinant plasmid. These lipase positive colonies would hydrolyze the castor oil triglycerides, thereby forming a clear zone around the colony. The number of recipients at the time of plating onto selective medium was determined by plating 100 µl aliquots of serial dilutions onto nutrient agar plates supplemented with 50 µg/ml rifampicin.

Transconjugant colonies, recipient cells which have received a recombinant pCP13 plasmid appeared at frequencies which ranged from $2 \times 10^{-6}$ to $2 \times 10^{-5}$ per recipient. Approximately 32,500 transconjugants, or approximately 2,500 transconjugants per experiment, containing recombinant plasmids from each of the E. coli clone pools grew on the selection plates.

Out of 2,375 transconjugants from the mating utilizing pool #13, 4 colonies produced clear zones indicating the presence of lipase activity. Similarly, 35 out of 2,850 transconjugants from the mating using pool #23 also produced clear zones. The transconjugant colonies exhibiting lipase activity were streaked onto the same agar plates containing castor oil and again clear zones (lipase) were formed in every strain.

Example 3

Transformation of Plasmids Containing a Lipase Gene and an Antibiotic Resistance Marker into E. coli

Part 1: Isolation of plasmids containing a lipase gene from P. oleovorans

Three different P. oleovorans strains containing lipase clones were grown in tubes containing 5 ml of nutrient broth supplemented with 10 µg/ml Rif and 5 µg/ml Tc. The tubes were incubated overnight in a roller drum at 30° C. Two 1.5 ml aliquots of cells were harvested by centrifugation in 1.5 ml capacity microcentrifuge tubes for 1 min at room temperature at 13,000 x g. The cells were resuspended with a sterile toothpick in 200 µl of a cold solution, which contained 0.05 M glucose (Sigma Chemical Co., St. Louis, MO), 0.01 M EDTA, 0.025 M Tris pH 8, and 4 mg/ml lysozyme and incubated for 5 min at room temperature. Cell lysis was accomplished by the addition of 400 µl of freshly made 0.2 N sodium hydroxide (NaOH) (Sigma Chemical Co., St. Louis, MO) and 1% (w/v) SDS and incubated at 4° C for 5 min. The proteins and other cellular debris were precipitated with the addition of 300 µl of cold 3 M sodium acetate (Sigma Chemical Co., St. Louis, MO) pH 4.8, incubation at 4° C for 5 min, and centrifugation for 10 min at 13,000 x g at 4° C. 700 µl of the supernatant was transferred to a fresh 1.5 ml capacity microcentrifuge tube and extracted with an equal volume of unbuffered (1:1, v/v) phenol/chloroform to remove any remaining contaminating proteins.

The nucleic acids in 500 $\mu$l of the extracted aqueous phase were precipitated by the addition of 2 vol of cold 100% ethanol. After incubation at -70°C for 25 min, the precipitated DNA was harvested by centrifugation at 13,000 x g for 20 min at 4°C. The nucleic acid pellets were washed 3 times with 70% ethanol to remove residual salts and phenol/chloroform. Then the pellets were dried in a Speed-Vac Concentrator for 7 min. The nucleic acids were resuspended in 50 $\mu$l of TE pH 8 buffer.

Part 2: Preparation of competent E. coli cells for transformation.

A tube containing 5 ml of Luria broth was inoculated with E. coli HB101 bacteria and grown in a roller drum overnight at 30°C. Then a flask containing 400 ml of Luria broth was inoculated with HB101 cells to an $A_{600}$ of 0.02 and incubated at 37°C on a rotary shaker at 200 rpm for 125 min. After incubating the cells at 4°C for 20 min, the bacteria were harvested by centrifugation at 2,000 x g for 15 min at 4°C. The harvested E. coli cells were prepared for transformation by the calcium chloride method of Silhavy et al., Experiments with Gene Fusions, Cold Spring Harbor Laboratory, NY (1984), p. 169.

Part 3: Transformation of plasmids into E. coli.

Ten microliters of the partially purified DNA prepared in Part 1 was transformed into the cells of E. coli HB101 by the method of Silhavy et al., Experiments with Gene Fusions, Cold Spring Harbor Laboratory, NY (1984), p. 170. The cells were grown on Luria agar plates containing 15 $\mu$g/ml Tc overnight at 37°C. This selection medium allows the growth of only the cells containing the recombinant pCP13 plasmid, which possessed a lipase gene and the Tc resistance marker.

Example 4

Physical Analysis of Recombinant pCP13 Plasmids Containing the P. sp. ATCC 21808 Lipase Gene

The recombinant pCP13 plasmids were partially purified from cultures of three different Lip[+] P. oleovorans strains as described in Example 3, Part 1. One-half (25 $\mu$l) of the partially purified plasmid DNA was mixed with 7.5 U of restriction enzyme XhoI in a 50 $\mu$l solution, which consisted of 0.025 M Tris-HCl pH 7.8, 0.05 M NaCl, 0.01 M $MgCl_2$, 100 $\mu$g/ml BSA, 2 mM $\beta$-mercaptoethanol, and 200 $\mu$g/ml ribo-nuclease A (RNase A), and incubated at 37°C overnight.

The digested plasmid DNA was analysed by 0.7% agarose gel electrophoresis. The gel and reservoir buffer was 0.089 M Tris-borate and 0.089 boric acid (Mallinckrodt Inc., Paris, KY). The gel (DNA) was stained at room temperature with 0.5 $\mu$g/ml ethidium bromide for 25 min and rinsed at room temperature for 30 min with 1 mM $MgSO_4$ to remove the non-DNA bound ethidium bromide. The DNA fragment pattern and sizes are shown in Table 1.

## TABLE 1

## SIZE ANALYSIS OF LIPASE POSITIVE RECOMBINANT PLASMIDS

| Frag. # | pHSH1 | pHSH2 | pHSH7 |
|---------|-------|-------|-------|
| 1 | | | 9.4 |
| 2 | 7.4 | | |
| 3 | 6.9 | 6.9 | 6.9 |
| 4 | 5.6 | 5.6 | 5.6 |
| 5 | | 4.9 | |
| 6 | 3.1 | 3.1 | |
| 7 | | | 1.2 |
| 8 | 0.7 | 0.7 | 0.7 |
| Total Kb | 23.7 | 21.2 | 23.8 |

Each of the three types of recombinant plasmids possessed a little over 20 Kb of Pseudomonas DNA. Three common fragments, which contain the lipase gene total 13.2 Kb and are underlined as shown in Table 1 above.

Example 5

Lipase Activity of P. oleovorans Strains Containing Recombinant Plasmids Possessing the Lipase Gene

Tubes containing 3 ml of peptone/yeast extract (PYE) medium (pH 7.5), which contains (grams per liter) 8 g of peptone, 2 g of yeast extract, and 5 g of NaCl was supplemented with 5 $\mu$g/ml rifampicin and 15 $\mu$g/ml Tc and then inoculated with P. oleovorans ATCC 8062 Rif$^r$ strains containing the lipase clones. Also, P. oleovorans ATCC 8062 Rif$^r$ that did not contain a recombinant clone and P. sp. ATCC 21808 Rif$^r$ served as controls, (negative and positive, respectively) and were grown in the above medium, but without Tc. Tubes were incubated in a roller drum at 30°C overnight. Subsequently, 500 ml capacity "baffled" Erlenmeyer flasks (flask bottom possesses 3 indentations) containing 50 ml of the above medium supplemented with 0.5% (w/v) olive oil (Sigma Chemical Co., St. Louis, MO) were inoculated to an $A_{420}$ of 0.08 and incubated at 30°C in a rotary shaker for 3 days at 30°C.

Culture aliquots (1 ml) were centrifuged at 13,000 x g for 1 min in an Eppendorf centrifuge. The lipase activity of the culture supernatants was determined in a spectrophotometric assay utilizing p-nitrophenyl palmitate (PNPP) as artificial substrate. The reaction volume was 1 ml and the reaction mixture contained 0.1 M Tris pH 7.5, 0.5% (v/v) Triton X-100 (Sigma Chemical Co., St. Louis, MO), and 0.2 mg/ml p-nitrophenyl palmitate (Sigma Chemical Co., St. Louis, MO). The lipase assay was initiated by adding an aliquot of culture supernatant to the reaction mixture and followed by the increase in absorbance at 400 nm for 1 min. The lipase mediated hydrolysis of PNPP, which released the p-nitrophenyl moiety which produces a yellow color. The lipase activities are shown in Table 2.

## TABLE 2

### LIPASE ACTIVITY OF SUPERNATANTS

| Strains | Lipase Act. (U/ml) |
|---|---|
| P. oleovorans ATCC 8062 Rif[r]: | |
| (no plasmid) | 0.00 |
| (lipase clone 1) | 0.017 |
| (lipase clone 2) | 0.017 |
| (lipase clone 3) | 0.010 |
| P. sp. ATCC 21808 Rif[r] | 18.5 |

The control P. oleovorans strain showed no lipase activity. On the other hand, the P. sp. ATCC 21808 possessed a high lipase activity. One unit of lipase activity is defined as the amount of lipase enzyme required to hydrolyze 1 $\mu$mole of PNPP in 1 min at 30° C in the assay conditions described above. The P. oleovorans strains containing the recombinant plasmids that possess a lipase gene had lipase activity that was approximately 0.1% of the level as compared to P. sp. ATCC 21808. This low but significant lipase activity is probably due to the inability of the transcription and/or translation machinery of P. oleovorans to recognize the regulatory (initiation) sequences of P. sp. ATCC 21808.

Example 6

Preparation and Isolation of a Recombinant Plasmid that Contains a Smaller P. sp. ATCC 21808 DNA Fragment that Possesses the Intact Lipase Gene

Part 1: Detection of a smaller common DNA fragment containing the lipase gene.

As described in Example 4, the three types of P. sp. ATCC 21808 DNA inserts which contain the lipase gene have 13.2 Kb of common DNA. The three types of gene have 13.2 Kb of common DNA. The three types of Lip[+] plasmid DNA was isolated as described in Example 1, Part 2. Then a mixture of 0.5 $\mu$g of each of the three types of recombinant plasmids and 10 U of restriction enzyme HindIII in a 20 $\mu$l solution which contained the same components as the digestion buffer as in Example 4, was incubated at 37° C for 1 h. The digested plasmid DNAs were analyzed by agarose gel electrophoresis. The three distinct DNA fragment patterns showed a single 8.0 Kb common DNA fragment which probably has the intact lipase gene.

Part 2: Isolation of the HindIII generated 8.0 Kb common fragment (called F2).

A mixture of 100 $\mu$g of plasmid pHSH1 DNA and 300 U of HindIII in a 200 $\mu$l solution which contained the same components as the digestion buffer as in Example 4 was incubated at 37° C for 1 h. Then the DNA digestion fragments were separated by vertical agarose gel electrophoresis. The agarose gel concentration was 0.9%. The gel and reservoir buffer was 0.04 M Trisacetate and 2 mM EDTA. The gel was stained and destained as described in Example 4. The gel containing the 8.0 Kb DNA band was excised with a razor blade and extruded through an 18-gauge needle into a tube containing 15 ml of distilled $H_2O$. The tube was rocked back and forth overnight at 4° C. The DNA solution containing the 8.0 Kb fragment was concentrated by multiple centrifugations to remove the agarose and multiple extractions with n-butanol to remove the $H_2O$. Finally, the DNA was precipitated and resuspended in TE pH 8 as described in Example 1, Part 3.

Part 3: Preparation of plasmid pCP13 for cloning.

A mixture of 60 μg of plasmid pCP13 and 180 U of restriction enzyme HindIII in a 250 μl solution which contained the same components as the digestion buffer as in Example 4 was incubated at 37°C overnight. The restriction enzyme and other contaminating proteins were removed with organic solvent extractions as described in Example 1, Part 3. Also the linearized pCP13 DNA was harvested by ethanol precipitation, washed 3 times with 70% ethanol, and resuspended as described in Example 1, Part 3. Analysis of the plasmid DNA by agarose gel electrophoresis showed that the digestion was complete.

HindIII digested pCP13 DNA was dephosphorylated, as described in Example 1, Part 4, to prevent plasmid recircularization in the subsequent ligation reaction. Moreover, the contaminating proteins were removed, and the dephosphorylated DNA was precipitated, harvested, washed, and resuspended in TE pH 8 as described in Example 1, Part 3.

Part 4: Recombining the F2 fragment with plasmid pCP13.

Equal amounts (0.5 μg) of F2 and pCP13 DNA and 20 U of T4 DNA ligase were combined in a 20 μl solution, which contained the same ligation buffer as described in Example 1, Part 4 and incubated at 14°C overnight. Analysis of the DNA ligation mixture by agarose gel electrophoresis showed that the ligation was complete.

Part 5: Isolation of E. coli cells containing the F2/pCP13 recombinant plasmid.

Competent E. coli HB101 cells were prepared as described in Example 3, Part 2. Then one-half of the ligated DNA prepared in the previous part was transformed into the cells of HB101 and the transformants were selected as described in Example 3, Part 3.

The plasmid content of 22 Tc$^r$ HB101 transformants were analyzed as described in Example 4. In this case, the partially purified plasmid DNA was digested with restriction enzyme HindIII. Analysis by agarose gel electrophoresis (0.9% agarose concentration) showed that all 22 transformants contained a recombinant pCP13 plasmid that possessed the 8.0 Kb F2 fragment.

Part 6: Determination that the F2 fragment contains an intact lipase gene.

The F2/pCP13 recombinant plasmid (called pHSH17) was transferred by conjugation from E. coli HB101 to P. oleovorans ATCC 8062 Rif$^r$ as described in Example 2, Part 2. All P. oleovorans Tc$^r$ transconjugant colonies formed clearing zones on the agar plates that contained translucent castor oil emulsions. The plasmid content of 4 P. oleovorans Tc$^r$ transconjugants were analyzed as described in the previous part. Analysis by agarose gel electrophoresis showed that all 4 transconjugants contained a recombinant pCP13 plasmid that possessed the 8.0 Kb F2 fragment. This result indicates that the F2 fragment contains an intact P. sp. ATCC 21808 lipase gene.

Example 7

Conjugation of a Plasmid Containing a Lipase Gene and an Antibiotic Resistance Marker into Pseudomonas aeruginosa and Pseudomonas fluorescens

The recombinant plasmid pHSH17, constructed as described in Example 6, Part 4 was used as the source of the DNA fragment (F2) which contains the lipase gene. Plasmid pHSH17 was transferred by conjugation from E. coli HB101 to P. aeruginosa PAO2003 (B. Holloway, Dept. of Genetics, Monash University, Clayton, Victoria, Australia) Rif$^r$ and P. fluorescens ATCC 948 Rif$^r$ as described in Example 2, Part 2. Since P. aeruginosa PAO2003 Rif$^r$ possesses a low indigenous lipase activity, transconjugants formed slightly larger clearing zones on castor oil agar plates (see Example 2, Part 2). Moreover, P. fluorescens transconjugants formed clearing zones. The plasmid content of several P. aeruginosa and P. fluorescens Tc$^r$ transconjugants were analyzed as described in Example 6, Part 5. Analysis by agarose gel electrophoresis showed that all transconjugants of both species contained plasmid pHSH17.

The lipase activity of P. aeruginosa, P. fluorescens, and P. oleovorans strains containing pHSH17 were determined as described in Example 5. As in Example 5, the strains that did not contain pHSH17 served as negative controls and were grown as described.

The lipase activities are shown in Table 3. The control P. oleovorans and P. fluorescens strains did not possess any lipase activity, while P. aeruginosa possessed a low lipase activity. The P. fluorescens - (pHSH17) recombinant strain possessed a low lipase activity that was slightly higher than the lipase activity of the P. oleovorans (pHSH17). On the other hand, P. aeruginosa (pHSH17) possessed a lipase activity that was approximately five times higher than the P. fluorescens strain's activity (after subtracting for background).

## TABLE 3

### LIPASE ACTIVITY OF DIFFERENT PSEUDOMONAS STRAINS

| Strain | Lipase Activity (U/ml supernatant) |
|---|---|
| P. oleovorans ATCC 8062 Rif$^r$ | |
| (no plasmid) | 0.00 |
| (pHSH17) | 0.03 |
| P. aeruginosa PAO2003 Rif$^r$ | |
| (no plasmid) | 0.19 |
| (pHSH17) | 0.40 |
| P. fluorescens ATCC 948 Rif$^r$ | |
| (no plasmid) | 0.00 |
| (pHSH17) | 0.04 |

Example 8

Preparation and Isolation of a P. aeruginosa Strain Containing a Lipase Gene In Recombinant Plasmids

Plasmid pME290 (Cb Km Mob⁻; D. Haas, Mikrobiologisches Institut, Eidgenossische Technische Hockschule, Zürich, Switzerland), which was derived from P. aeruginosa plasmid pVS1 (HgSuMob IncP-(unclassified) Itoh et al., Plasmid (1984) 11:206-220) is 6.8 Kb in size, carries genes encoding carbenicillin (Cb) and Km resistance, and possesses several sites for cloning foreign DNA via insertional inactivation (Itoh and Haas, Gene (1985) 36:27-36). Moreover, the non-mobilizable plasmid pME290 can be introduced by transformation and maintained in various species of pseudomonads at a level between 7-10 copies per cell.

The HindIII generated 8.0 Kb F2 DNA fragment (isolated as described in Example 6, Part 2) contained the lipase gene.

Plasmid pME290 was purified from cells of P. aeruginosa PAO2003 Rif$^r$ (pME290) by the methods described in Example 1, Part 2. The bacteria were grown in nutrient broth supplemented with 5 g/l of yeast extract and 100 $\mu$g/ml of carbenicillin (Sigma Chemical Co., St. Louis, MO).

17.5 $\mu$g of plasmid pME290 DNA and 52.5 U of restriction enzyme HindIII were combined in a 250 $\mu$l solution which contained the same components as the digestion buffer used in Example 4 and incubated at 37° C overnight. The restriction enzyme and other contaminating proteins were removed and the digested plasmid DNA was harvested, and resuspended as described in Example 1, Part 3. Then the digested pME290 DNA was dephosphorylated, as described in Example 1, Part 4. Moreover, the contaminating proteins were removed, and the dephosphorylated DNA was precipitated, harvested, washed, and resuspended in TE pH 8 buffer as described in Example 1, Part 3.

Equal amounts (0.5 $\mu$g) of DNA fragment F2 and pME290 DNA were ligated together as described in Example 6, Part 4. Analysis of the DNA ligation mixture by agarose gel electrophoresis showed that the ligation was complete.

Competent P. aeruginosa PA02003 Rif[r] cells for effective transformation were prepared by the method of Lederberg and Cohen, J. Bacteriology (1974) 119:1072-1074, except that the cells were grown in Luria broth at 37° C on a rotary shaker at 175 rpm for 3 h before $MgCl_2$ treatment.

For transformation of plasmids into P. aeruginosa, one-half of the DNA ligation mixture prepared above was transformed into the competent cells of P. aeruginosa PA02003 Rif[r] by the method of Lederberg and Cohen, J. Bacteriology (1974) 119:1072-1074. The cells were cultivated on nutrient agar plates containing 500 μg/ml carbenicillin overnight at 37° C. This selection medium allows the growth of only the cells containing the pME290 plasmid, which possessed the Cb resistance marker. To determine the number of cells containing recombinant DNA, the Cb[r] cells were screened for sensitivity to kanamycin. Since insertion of a fragment of DNA into the unique HindIII restriction site of pME290 inactivates the plasmid gene encoding for Km resistance, the frequency of Km sensitivity gives the number of P. aeruginosa cells containing a recombinant pME290 plasmid in the population of Cb[r] transformants. 2.30% of the Cb[r] resistant cells were also Km[s], indicating pME290 plasmids containing fragment F2 (called pHES3).

The lipase activity of the P. aeruginosa (pHES3) strain was determined as described in Example 5. The P. aeruginosa strain that contained the plasmid pME290 and P. sp. ATCC 21808 Rif[r] served as negative and positive controls (respectively) and were grown as described in Example 5. The lipase activities are shown in Table 4. The control P. aeruginosa (pME290) possessed a low indigenous lipase activity. The P. aeruginosa (pHES3) recombinant strain possessed lipase activity that was two times higher than the activity of the P. aeruginosa (pME290) (after subtracting for background). Moreover, the lipase activity of P. aeruginosa (pHES3) was approximately 5% of the activity of P. sp. ATCC 21808 Rif[r].

## TABLE 4

### LIPASE ACTIVITY OF P. AEURGINOSA (pHES3)

| Strain | Lipase Activity (U/ml supernatant) |
|---|---|
| P. aeruginosa PAO2003 Rif[r] | |
| (pME290) | 0.193 |
| (pHES3) | 0.661 |
| P. sp. ATCC 21808 Rif[r] | 8.30 |

Example 9

Construction of a New Recombinant Plasmid Containing DNA Fragment F2 and Subsequent Transformation into P. aeruginosa

The HindIII generated 8.0 Kb F2 DNA fragment (isolated as described in Example 6, Part 2) contained the lipase gene.

pME285 (HgKmMob[+]; D. Haas, Mikrobiologisches Institute, Eidgenössische Technische Hochschule, Zürich, Switzerland) was selected as the cloning vector because it was derived from the same P. aeruginosa plasmid (pVS1) as pME290 and it also carried a selectable mercury resistance (Hg) gene.

Plasmid pME285 (Itoh and Haas, Gene (1985) 36:27-36) was purified from cells of P. aeruginosa PA02003 Rif[r] (pME285) as described in Example 8, except that the bacterial growth medium was supplemented with 2.5 μg/ml mercuric chloride ($HgCl_2$) (Sigma Chemical Co., St. Louis, MO) and 100 μg/ml Km. Ten μg of plasmid pME285 DNA and 30 U of restriction enzyme HindIII were combined in a 125 μl solution which contained the same components as the digestion buffer used in Example 4.

After incubating the reaction at 37°C overnight, the digested pME285 plasmid was dephosphorylated as described in Example 1, Part 4, except the final volume was 216 μl. Moreover, the contaminating proteins were removed, and the dephosphorylated DNA was precipitated, harvested, washed, and resuspended in TE pH 8 as described in Example 1, Part 3.

Fragment F2 was ligated to plasmid pME285 as described in Example 8. One half of the DNA ligation mixture was transformed into competent cells of P. aeruginosa PAE2003 Rif$^r$ as described in Example 8. In this case, the transformed cells were cultivated on nutrient agar plates containing 50 μg/ml Rif and 30 μg/ml HgCl$_2$ for 2 days at 30°C. This selection medium allows the growth of only the cells containing the pME285 plasmid, which contains the Hg resistance genes. To determine the number of cells containing recombinant DNA, the Hg$^r$ cells were screened for sensitivity to Km. Since insertion of a fragment of DNA to the unique HindIII restriction site of pME285 inactivates the plasmid gene encoding for Km$^r$, Km sensitivity indicates a P. aeruginosa cell that harbors a recombinant plasmid. All Hg$^r$ transformants were Km$^s$, indicating that they have pME285 plasmids containing F2 (pHSH43).

Example 10

Transformation of a Plasmid Containing a Lipase Gene and an Antibiotic Resistance Marker in P. sp. ATCC 21808

Since P. sp. ATCC 21808 Rif$^r$ is naturally very sensitive to Hg, the recombinant plasmid pHSH43 (F2/pME285) was selected as the source of DNA that contained the lipase gene for transformation. Plasmid pHSH43 was partially purified from cells of P. aeruginosa PA02003 Rif$^r$ (pHSH43) as described in Example 3, Part 1.

The bacterial growth medium was nutrient broth supplemented with 5 μg/ml Rif and 2.5 μg/ml HgCl$_2$. 75 μl of the resuspended partially purified pHSH43 plasmid DNA was transformed into competent cells of P. sp. ATCC 21808 Rif$^r$ as described in Example 8, except the cells were grown in nutrient broth supplemented with 5 μg/ml Rif at 30°C on a rotary shaker at 200 rpm for 3 h before MgCl$_2$ treatment. P. sp. ATCC 21808 Rif$^r$ transformants containing the pHSH43 plasmid were selected and verified as described in Example 9.

Example 11

Lipase Activity of a P. sp. ATCC 21808 Strain Containing a Recombinant Plasmid Possessing a Lipase Gene

P. sp. ATCC 21808 Rif$^r$ (pHSH43) was grown as described in Example 5, except the growth medium was supplemented with 5 μg/ml HgCl$_2$. Also, a P. sp. ATCC 21808 Rif$^r$ strain that did not contain the plasmid and a strain that contained plasmid pME285 served as negative controls. The strain that did not contain the plasmid and the pME285 containing strain were grown in a medium that had 5 μg/ml Rif only and 5 μg/ml Rif + 2.5 μg/ml HgCl$_2$ + 100 μg/ml Km, respectively.

Quantitative lipase activity determinations were performed as described in Example 5. The lipase activities are shown in Table 5. P. sp. ATCC 21808 Rif$^r$ (pHSH43) possessed a maximum lipase activity that was 17 and 19 times higher than the strain that did not contain the plasmid and pME285-containing strain.

## TABLE 5

### LIPASE ACTIVITY OF P. SP. 21808 RIF^r STRAINS

| Strain | Lipase Activity (U/ml supernatant) | | |
|---|---|---|---|
| | 24 h | 48 h | 72 h |
| 21808 | 6.11 | 7.88 | 6.64 |
| 21808 (pME285) | 4.86 | 8.78 | 8.94 |
| 21808 (pHSH43) | 129 | 152 | 141 |

Example 12

Transformation of Two Different Recombinant Plasmids Each Containing the Lipase Gene into One Strain of P. sp. ATCC 21808

Part 1: Construction of a recombinant plasmid that could be maintained with pHSH43 in P. sp. ATCC 21808.

P. sp. ATCC 21808 Rif^r strain possessing two compatible plasmids (each containing the lipase gene) was constructed to increase lipase production.

The DNA insert containing the lipase gene was a 6.5 Kb subfragment of F2. F2 DNA (4.25 μg) and 12.75 U of restriction enzyme BamHI (New England Biolabs, Beverly, MA) were mixed together in a 50 μl solution which contained the same digestion buffer as described in Example 1, Part 3 (except 0.1 M NaCl was used) and incubated at 37° C overnight. Then the proteins were removed, and the digested DNA was harvested, washed, and resuspended in TE pH 8 as described in Example 1, Part 3.

Plasmid pCP13 was selected as the cloning vector, because it is derived from a plasmid which has a different incompatibility group than pME285's incompatibility group. Plasmid pCP13 DNA (20 μg) and 60 U of restriction enzyme HindIII were mixed together in a 125 μl solution, which contained the same digestion buffer as described in Example 1, Part 3, and incubated at 37° C for 1 h. After adjusting the solution to 0.1 M NaCl, 60 U of restriction enzyme BamHI was added and the digestion was incubated at 37° C overnight. Then the digested pCP13 DNA was dephosphorylated, the contaminating proteins removed, and the plasmid DNA was precipitated, harvested, washed, and resuspended in TE pH 8 as described in Example 9.

Subfragments of F2 were ligated to plasmid pCP13 as described in Example 8. The DNA ligation mixture was transformed into competent cells of E. coli HB101 as described in Example 3, Parts 2 and 3. The transformed cells were cultivated on Luria agar plates containing 15 μg/ml Tc and incubated at 37° C overnight. This selection medium allows the growth of only the cells containing the pCP13 plasmid, which contains the Tc resistance genes. To determine the number of cells containing recombinant DNA, the Tc^r cells were screened for sensitivity to Km. Since the insertion of a DNA fragment into the asymmetrical HindIII/BamHI site of pCP13 forms a plasmid that lacks the kanamycin resistance genes, Km^s indicates an E. coli cell that harbors a recombinant plasmid. All Tc^r transformants were Km^s.

The plasmid content of 12 Tc^rKm^s transformants were analyzed by agarose gel electrophoresis as described in Example 4. In this case, the partially purified plasmid DNAs were digested with HindIII and BamHI in 2-step digestion reactions as described above. One transformant contained a recombinant pCP13 plasmid that possessed the 6.5 Kb fragment (called pHSH31). Plasmid pHSH31 was transferred to P. oleovorans as described in Example 3, Part 2. P. oleovorans (pHSH31) produced clearing zones on the selection plates.

Since P. sp. ATCC 21808 is naturally highly resistance to Tc, a selectable antibiotic resistance marker was introduced into pHSH31 via the insertion of transposon Tn5 (which encodes for Km[r]) by the method of Ruvkun and Ausubel, Nature (1981) 289:85-88, with some modifications. The defective lambda virus containing Tn5 was a gift from R. Maier, Dept. of Biology, The Johns Hopkins Univ., Baltimore, MD. E. coli HB101 harboring plasmid pHSH31 was grown in tryptone broth as described in Example 1, Part 6. The multiplicity of infection was 0.5. Tn5 transposition events into the genome were selected by cultivating the bacteria on Luria agar plates containing 15 $\mu$g/ml Tc and 40 $\mu$g/ml Km. Tn5 tranposition events into plasmid pHSH31 were identified by isolating the plasmid DNA from several thousand Tc[r]Km[r] colonies (as described in Example 1, Part 2). Then the plasmid DNA was transformed into HB101 as described in Example 3, Parts 2 and 3. The transformed cells were cultivated on Luria agar plates containing 15 $\mu$g/ml Tc and 40 $\mu$g/ml Km and incubated at 37°C overnight. This selection medium allows the growth of only the cells which possess a pHSH31 plasmid with a Tn5 insertion (pHSH31::Tn5). Since Tn5 is 5.7 Kb and does not contain an EcoRI site, Tn5 insertions into the pCP13 part of pHSH31 were identified by agarose gel electrophoresis (as described in Example 4) by the appearance of a linearized pCP13 plasmid band that was 5.7 Kb larger. Eighteen out of 23 Tc[r]Km[r] transformants contained pHSH31 with Tn5 inserted into pCP13. One transformant was selected for this study (called pHSH31::Tn5-6).

Part 2: Conjugation of pHSH31::Tn5-6 into P. sp. ATCC 21808 (pHSH43).

Plasmid pHSH31::Tn5-6 was transferred from E. coli HB101 to P. sp. ATCC 21808 Rif[r] (pHSH43) by conjugation as described in Example 2, Part 2. The donor cells were cultivated in Luria broth containing 15 $\mu$g/ml Tc and 40 $\mu$g/ml Km. P. sp. ATCC 21808 Rif[r] Hg[r] transconjugants were selected on nutrient agar plates supplemented with 50 $\mu$g/ml Rif, 30 $\mu$g/ml HgCl$_2$, and 500 $\mu$g/ml Km. Plasmid analysis by agarose gel electrophoresis verified the presence of both plasmids. This strain construction shows that P. sp. 21808 can grow and simultaneously maintain two different chimeric plasmids containing lipase genes.

Part 3: Effect of olive oil concentration on lipase activity.

Furthemore, higher olive oil concentrations promoted higher expression of lipase activity. Shake flask cultures were grown as described above, except 250 $\mu$l (0.05% v/v) of olive oil was added to each culture after 24 h of incubation. As shown below and in Table 2, although strain 21808 (pHSH43) possessed a maximum lipase activity that was higher by approximately the same amount as the previous experiment (19 times higher than 21808 pME285 and 16 times higher than 21808), strain 21808 (pHSH43) possessed a maximum lipase activity that was 2.4 times higher (359 U/ml at 168 h) than in the previous experiment.

## TABLE 6

### LIPASE ACTIVITY OF P. SP. ATCC 21808 STRAINS
### SUPPLEMENTED WITH OLIVE OIL

| Strain | Lipase Activity (U/ml supernatant) | | | | |
|---|---|---|---|---|---|
| | 24 h | 48 h | 72 h | 96 h | 168 h |
| 21808 | 2.27 | 2.92 | 13.0 | 21.2 | 22.3 |
| 21808 (pME285) | 1.99 | 8.89 | 8.15 | 17.0 | 18.5 |
| 21808 (pHSH43) | 61.7 | 46.3 | 72.7 | 294 | 359 |

The higher lipase activity was not due to an increased cell number, since the viable cell concentration was the same in both experiments.

Example 13

Construction of a High Copy Number Recombinant Plasmid which Contains the Lipase Gene and Subsequent Transformation in P. sp. ATCC 21808

The DNA insert containing the lipase gene was a 2.5 Kb subfragment of F2. F2 DNA (4.25 μg) and 10 U of restriction enzyme BglII (New England Biolabs, Beverly, MA) were mixed together in a 37.5 μl solution, which contained the same digestion buffer as described in Example I, Part 3 and incubated at 37° C overnight. After adjusting the solution to 0.1 M NaCl, 10 U of restriction enzyme EcoRI was added and incubated at 37° C for 1 h. The proteins were removed, and the digested DNA was harvested, washed, and resuspended in TE pH 8 as described in Example 1, Part 3.

Plasmid pKT231 (K. Timmis, Department of Medical Biochemistry, University of Geneva, Geneva, Switzerland) was chosen as the cloning vector, because it possessed a host range of a wide variety of Gram-negative bacteria, a copy number of 15-20 in E. coli, a selectable kanamycin resistance (Km) gene, and was mobilization positive [Bagdasarian et. al., Gene, 16, 237-247 (1981)].

Plasmid pKT231 was purified from cells of E. coli containing pKT231 as described in Part 2, Example 1, except that the bacterial growth medium was supplemented with 25 μg/ml Km and 25 μg/ml streptomycin (Sm) (Sigma Chemical Co., St. Louis, MO). 3 μg of plasmid pKT231 DNA and 10 U of restriction enzyme BglII were combined in a 50 μl solution of digestion buffer described in Example 4. After incubating the reaction at 37° C overnight, the solution was adjusted to 0.1 M NaCl, 10 U of restriction enzyme EcoRI was added, and the digestion reaction was incubated at 37° C for an additional hour. Then the double digested pKT231 DNA was dephosphorylated, the contaminating proteins removed, and the plasmid DNA was precipitated, harvested, washed, and resuspended in TE pH 8 as described in Example 9.

Subfragments of F2 DNA were ligated to plasmid pKT231 as described in Example 8. The DNA ligation mixture was transformed into competent cells of E. coli HB101 as described in Example 3, Parts 2 and 3. The transformed cells were cultivated on Luria agar plates containing 50 μg/ml Km and 50 μg/ml Sm and incubated at 37° C overnight. This selection medium allowed the growth of only the cells containing the pKT231 plasmid, which contained both the Km and Sm resistance genes. To identify the strain(s) containing the desired 2.5 Kb DNA insert, plasmid content of Km$^r$ Sm$^r$ transformants were analyzed by agarose gel electrophoresis as described in Example 4. In this case, the partially purified plasmid DNAs were digested with restriction enzymes BglII and EcoRI in 2-step digestion reactions as described above. Ten of twenty transformants contained a recombinant pKT231 plasmid that possessed the 2.5 Kb fragment (called pHES8).

Plasmid pHES8 was transferred to P. sp. ATCC 21808 by transformation as described in Example 10. Plasmid pHES8 was partially purified from cells of E. coli HB101 (pHES8) as described in Example 3, Part 1. The medium used to cultivate the E. coli HB101 (pHES8) cells was Luria broth supplemented with 50 μg/ml Km and 50 μg/ml Sm. 192 μl of the partially purified pHES8 plasmid DNA was transformed into competent cells of P. sp. ATCC 21808 Rif$^r$ as described in Example 10. The transformed cells were cultivated on nutrient agar plates containing 500 μg/ml Km and 500 μg/ml Sm. This selection medium allowed the growth of only the cells containing the pHES8 recombinant plasmid. P. sp. ATCC 21808 Rif$^r$ Km$^r$ Sm$^r$ transformants containing the pHES8 plasmid were selected and verified as described above.

The lipase activity of P. sp. 21808 Rif$^r$ (pHES8) was determined as described in Example 5, except the growth medium was supplemented with 50 μg/ml Km. Morever, P. sp. ATCC 21808 Rif$^r$ strain that did not contain the plasmid and a strain that contained plasmid pKT231 served as negative controls. The strain that did not contain the plasmid and the pKT231 containing strain were grown in a medium that had 5 μg/ml Rif only and 5 μg/ml Rif + 50 μg/ml Km respectively.

Quantitative lipase activity determinations were performed as described in Example 5. The lipase activities are shown in Table 7. P. sp. ATCC 21808 Rif$^r$ (pHES8) possessed a lipase activity that was 42 and 62 times higer than the strain that did not contain the plasmid and the pKT231-containing strain.

18

## TABLE 7

### LIPASE ACTIVITY OF P. SP. 21808 RIF$^r$ STRAINS

| Strain | Lipase Activity (U/ml of culture) |
|---|---|
| 21808 | 18.9 |
| 21808 (pKT231) | 12.8 |
| 21808 (pHES8) | 800 |

It is evident from the above results that high levels of lipase production can be achieved by employing constructs of the subject invention. Thus, using endogenous or exogenous transcription initiation regions, commercially desirable levels of lipase can be secreted into the supernatant and continuously produced and isolated. Levels can be further enhanced by employing combinations of plasmids, particularly high copy number plasmids, where the two plasmids may be compatibly maintained in the same host, using strong promoters, integration and amplification of the gene and the like. Of particular interest is the use of a pseudomonad host having one or a plurality of plasmids, where the lipase gene is expressed and secreted.

All publications and patent applications mentioned in this specification are indicative of the level of skill of those skilled in the art to which this invention pertains. All publications and patent applications are herein incorporated by reference to the same extent as if each individual publication or patent application was specifically and individually indicated to be incorporated by reference.

Although the foregoing invention has been described in some detail by way of illustration and example for purposes of clarity of understanding, it will be obvious that certain changes and modifications may be practiced within the scope of the appended claims.

EXAMPLE 14

Construction of a Recombinant Plasmid Which Contains the Lipase Gene Fused to the tac Promoter and Subsequent Conjugation into P. sp. AtCC 21808

Part 1. Construction of a recombinant plasmid containing an intact lipase gene

The DNA insert containing the lipase gene was a 5.5 kb subfragment of F2. Plasmid pHSH17 DNA (20 ug), which contained F2 and 20 U of restriction enzyme HindIII were mixed together in a 20 ul solution, which contained the same digestion buffer as described in Example I, Part 3, and incubated at 37°C for 1 h. After adjusting the solution to 0.1M NaCl, 40 U of restriction enzyme EcoRI was added and incubated at 37°C for 1 h. The proteins were removed, and the digested DNA was harvested, washed, and resuspended in TE ph 8 as described in Example 1, Part 3.

The cloning vector, plasmid pBR322 DNA (20 ug) was digested with restriction enzymes HindIII and EcoRI in a 2-step digestion reaction as described above. The proteins were removed, and the digested DNA was harvested, washed, and resuspended as described in Example 1, Part 3.

Subfragments of F2 DNA generated from the digestion of plasmid pHSH17 were ligated to plasmid pBR322 as described in Example 6, Part 4. The DNA ligation mixture was transformed into competent cells of E. coli HB101 as described in Example 3, Parts 2 and 3. The transformed cells were cultivated on Luria agar plates containing 50 ug/ml ampicillin and incubated at 37°C overnight. This selection medium allowed the growth of only the cells containing the pBR322 plasmid, which contained the ampicillin resistance genes. To identify the strain(s) containing the desired 5.5 kb DNA insert, plasmid content of Tc$^r$ transformants was analyzed by agarose gel electrophoresis as described in Example 4. In this case, the partially purified plasmid DNAs were digested with restriction enzymes HindIII and EcoRI in 2-step digestion reactions as described above. One of eighteen transformants contained a recombinant pBR322 plasmid that possessed the 5.5 kb fragment (called pHRA1).

19

Part 2: Deletion of the lipase gene promoter

Plasmid pHRA1 possessed a unique BglII restriction site located near the lipase gene promoter region. An assortment of delections of the lipase promoter region was constructed by progressive digestion of plasmid pHRA1 with the exonuclease Bal31 (International Biotechnologies, Inc., New Haven, CT) from the unique BglII restriction site. Bal31 is an exonuclease which deletes nucleotides from both the 3' and 5' ends of linear DNA fragments. Plasmid pHRA1 DNA (80 ug) and 128 U of restriction enzyme BglII were mixed together in a 45 ul solution, which contained the same digestion buffer as described in Example I, Part 3, and incubated at 37°C overnight. The proteins were removed, and the digested DNA was harvested, washed, and resuspended in TE ph 8 as described in Example 1, Part 3.

Eight aliquots containing 10 ug of BglII digested pHRA1 DNA and 100 U of Bal31 Fast were mixed together in a 100ul solution, which contained 600mM NaCl, 12.5 mM CaCl$_2$, 12.5mM MgCl$_2$, 20mM Tris-HCl ph 8, and 1mM EDTA and incubated for 45 min at 30°C. The proteins were removed, and the digested DNA was harvested, washed, and resuspended in TE ph 8 as described in Example 1, Part 3.

Part 3: Preparation of DNA fragments containing deletions of the lipase gene promoter for fusion to the tac promoter

The Bal31-treated pHRA1 DNA was blunt-ended by adding 5 U of E. coli DNA polymerase (Klenow Fragment; New England Biolabs, Beverly, MA) in a 260 ul solution, which contained 25mM Tris-HCl pH7.8, 50mM NaCl, 10mM MgCl$_2$, 100 ug/ml bovine serum albumin, .1mM dATP, dTTP, dCTP, and dGTP, and 1mM dithiothreitol and incubated at 37°C for 1.5 h. The proteins were removed, and the digested DNA was harvested, washed, and resuspended in TE pH 8 as described in Example 1, Part 3.

Multiple EcoRI linkers (GGAATTCC; Stratagene, La Jolla, CA) were added to the ends of the blunt-ended pHRA1 DNA by adding 2000 U of T$_4$ DNA ligase to a 200ul solution, which contained 25mM Tris-HCl pH 7.8, 10mM MgCl$_2$, 4mM β-mercaptoethanol, 0.4mM ATP and 5 ng/ml EcoRI linkers and incubated at room temperature overnight.

EcoRI linkered plasmid pHRA1 DNA and 100 U of restriction enzyme EcoRI were mixed together in a 300 ul soltuion, which contained the same digestion buffer as described in Example 13 and incubated at 37°C for 6h. The proteins were removed, and the digested DNA was harvested, washed, and resuspended in TE ph 8 as described in Example 1, Part 3.

The DNA fragments that contained lipase genes with possible deletions in the promoter region were separated from the pBR322 plasmid fragments by vertical agarose gel electrophoresis as described in Example 6, Part 2.

Part 4: Construction of the recombinant plasmid containing the tac promoter fused to the lipase gene

Plasmid pMMB22 (M. Bagdasarian, Michigan Biotechnology Institute, Lansing, MI) was chosen as the cloning vector, because it possessed a tac promoter, a host range of a wide variety of Gram-negative bacteria, a selectable ampicillin resistance (Ap) gene, and mobilization genes [Bagdasarian et.al., Gene, 26, 273-282 (1983)]. Moreover, this plasmid was used successfully to overexpress the P. aeruginosa phosphomannose isomerase gene in P. aeruginosa.

Plasmid pMMB22 was purified from cells of E. coli containing pMMB22 as described in Part 2, Example 1, except that the bacterial growth medium was supplemented with 25 ug/ml Ap (Sigma Chemical Co., St. Louis, MO). 44 ug of plasmid pMMB22 DNA and 40 U of restriction enzyme EcoRI were combined in a 125 ul solution of digestion buffer as described in Example 13 and incubated at 37°C overnight. EcoRI digested pMMB22 DNA was dephosphorylated, as described in Example 1, Part 4, to prevent plasmid recircularization in the subsequent ligation reaction. The contaminating proteins were removed, and the dephosphorylated DNA was precipitated, harvested, washed, and resuspended in TE pH 8 as described in Example 1, Part 3. The purified DNA fragments that contained lipase genes with possible deletions in the promoter region (described in Part 3 above) were ligated to plasmid pMMB22 as described in Example 8.

Part 5: Transformation of plasmids into E. coli

The DNA ligation mixture was transformed into competent cells of E. coli DH5 as described in Example 3, Parts 2 and 3. The transformed cells were cultivated on Luria agar plates containing 50 ug/ml Ap and incubated at 37°C overnight. This selection medium allowed the growth of only the cells containing pMMB22 plasmid, which contained the Ap resistance gene. To identify the strain(s) containing the desired DNA insert that possessed a promoterless lipase gene fused to the tac promoter, the Ap$^r$ transformants were screened on Luria agar plates supplemented with 50 ug/ml Ap, 0.05% castor oil, and either with or without 1mM isopropyl-thio-galactoside (1PTG; International Biotechnologies Inc., New Haven, CT). One DH5 Ap$^r$ transformant produced a large clearing zone only when 1PTG was present. Genes controlled by the tac promoter are fully induced by the non-metabolizable 1PTG molecule. This DH5 strain contained a recombinant pMMB22 plasmid that possessed the tac promoter fused to the lipase gene (called pHES12).

Part 6: Introduction of plasmid pHES12 into P. sp. ATCC 21808

Plasmid pHES12 was transferred to P. sp. ATCC 21808 by conjugation as described in Example 2, Part 2. P. sp. ATCC 21808 transconjugants were selected on nutrient agar plates with 50 ug/ml Rif and 1000 ug/ml Ap.

Part 7: Lipase activity of P. sp. ATCC 21808 (pHES12) strains

The lipase activity of P. sp. ATCC 21808 Rif$^r$ (pHES12) was determined as described in Example 5, except the growth medium was supplemented with 50 ug/ml Ap with and without 1mM 1PTG. Moreover, the P. sp. ATCC 21808 Rif$^r$ strain that did not contain the plasmid and a strain that contained plasmid pMMB22 served as negative controls. The strain that did not contain the plasmid and the pMMB22 containing strain were grown in a medium that had 5 ug/ml Rif with and without 1mM 1PTG only and 5 ug/ml Rif + 50 ug/ml Ap with and without 1PTG respectively.

Quantitative lipase activity determinations were performed as described in Example 5. The lipase activities are shown in Table 1.

## TABLE 1

### LIPASE ACTIVITY OF P. sp. 21808 RIF$^r$ STRAINS

| STRAIN (U/ml of culture) | 1mM 1PTG | LIPASE ACTIVITY |
|---|---|---|
| 21808 | – | 12.6 |
| 21808 | + | 12.6 |
| 21808 (pMMB22) | – | 11.7 |
| 21808 (pMMB22) | + | 13.4 |
| 21808 (pHES12) | – | 26.4 |
| 21808 (pHES12) | + | 1070 |

P. sp. ATCC 21808 Rif$^r$ (pHES12) when grown without 1PTG possessed a lipase activity that was only 2 times higher than the strain that did not contain the plasmid or contained the parental plasmid. On the other hand, grown in the presence of 1PTG, P. sp. ATCC 21808 Rif$^r$ (pHES12) possessed a lipase activity that was 85 times higher than both control strains.

EXAMPLE 15

Construction of a Recombinant Plasmid Which Contains the Lipase Gene Fused to the xyl Promoter and Subsequent Conjugation into P. sp. ATCC 21808

Part 1: Construction of the recombinant plasmid containing the xyl promoter fused to the lipase gene

Plasmid pNM185 (K. Timmis, Department of Medical Biochemistry, University of Geneva, Geneva, Switzerland) was chosen as the cloning vector, because it possessed a xyl promoter, a host range of a wide variety of Gram-negative bacteria, a selectable kanamycin resistance (Km) gene, and mobilization genes [Mermod et.al., Journal of Bacteriol., 167, 447-454 (1986)]. Moreover, this plasmid was used successfully to overexpress the xyl E gene in a wide variety of Gram-negative bacteria.

Plasmid pNM185 and pHES12 was purified from cells of E. coli containing pNM185 and pHES12 as described in Part 2, Example 1, except that the bacterial growth medium was supplemented with 50 ug/ml Km. Plasmid pNM185 was digested and dephosphorylated and plasmid pHES12 was digested as described in Example A, Part 4. The DNA fragment from pHES12 which contained a promoterless lipase gene was ligated to plasmid pNM185, which contained the xyl promoter as described in Example 8.

Part 2: Transformation of the recombinant plasmid into E. coli

The DNA ligation mixture was transformed into competent cells of E. coli DH5 as described in Example 3, Parts 2 and 3. The transformants were selected as described in Example A, Part 5, except 50 ug/ml Km was used. To identify the strains containing the desired orientation of the DNA insert to the xyl promoter, the Km$^r$ transformants were screened on Luria agar plates supplemented with 50 ug/ml Km, 0.05% castor oil, and either with or without 5mM m-toluic acid (m-tol; Eastman Kodak, Rochester, NY). Several Km$^r$ transformants produced large clear zones only when m-tol was present. Genes controlled by the m-tol molecule. These pH5 strains contained a recombinant pNM185 plasmid that possessed the xyl promoter fused to the promoterless lipase gene (called pHES12X).

Part 3: Introduction of pHES12X into P. sp. ATCC 21808

Plasmid pHES12X was transferred to P. sp. ATCC 21808 by conjugation as described in Example A, Part 6, except 500 ug/ml km was used.

Part 4: Lipase activity of P. sp. ATCC 21808 (pHES12X) strains

The lipase activity of P. sp ATCC 21808 Rif$^r$ (pHES12X) was determined as described in Example A, Part 7, except 50 ug/ml km and 0.1mM m-tol was used. Quantitative lipase activity determinations were performed as described in Example 5. The lipase activity determinations were performed as described in Example 5. The lipase activity of 21808 (pHES12X) was 750 U/ml or approximately 60 times greater than the strain without the plasmid **or the strain with the parental** plasmid.^@^@^@^@^@^@^@^@^@^@^@^@^@^@^@^@^@^@^@^@^@^@-
^@^@^@^@^@
^@^@^@^@^@^@^@^@^@^@^@^@^@^@^@^@^@^@^@^@^@^@^@-
^@^@^@^@^@^@^@
^@^@^@^@^@^@^@^@^@^@^@^@^@^@^@^@^@^@

DEPOSIT OF MICROORGANISMS

Living cultures of plasmid pHES8 in ATCC 21808 assigned the ATCC Designation 68160, plasmid pHES12 in ATCC 21808 assigned the ATCC Designation 68161, and plasmid pHES12X in ATCC 21808 assigned the ATCC Designation 68181 have been accepted for Deposit under the Budapest Treaty on the International Recognition of the Deposit of Microorganisms for the purposes of patent procedure by the American Type Culture Collection, 12301 Parklawn Drive, Rockville, MD 20852.

**Claims**

1. A Pseudomonas cell comprising a chromosomal lipase gene and at least one multicopy vector comprising a lipase gene capable of expression of lipase in said cell, wherein said lipase is characterized by being derived from the strain ATCC 21808.

2. A Pseudomonas cell according to Claim 1, wherein said vector is pHES3, pHSH43, pHSH31, pHES12 or pHES12X.

3. A plasmid comprising a P incompatibility replication system, a Pseudomonas lipase gene characterized by being derived from the strain ATCC 21808, and a marker for selection.

4. A plasmid according to Claim 3, wherein said plasmid is pHES3, pHSH43 pHSH31, pHES12 or pHES12X.

5. A method for producing lipase, wherein said lipase is characterized by being derived from the strain ATCC 21808, said method comprising:
   growing cells of a Pseudomonas strain comprising a genomic lipase gene and multiple copies of a Pseudomonas lipase gene encoding said lipase for a time sufficient and under conditions for said lipase to be expressed; and
   harvesting said lipase free of said cells.

6. A method according to Claim 5, wherein said multiple copies are components of at least one vector.

7. A method according to Claim 5, wherein said vector is pHES3, pHSH43, pHSH31, pHES12 or pHES12X.

8. A method according to Claim 5, wherein said cells produce at least five fold greater amount of lipase than said cells in the absence of said multicopy lipase gene.

9. A method according to Claim 5, wherein said cells comprise two multicopy vectors, each of which vectors comprise a Pseudomonas lipase gene encoding said lipase.

10. A method for producing lipase, said method comprising:
    growing cells of Pseudomonas strain ATCC 21808 comprising plasmids pHSH43 and pHSH31 for a time sufficient and under conditions for said lipase to be expressed; and
    harvesting said lipase free of said cells.

11. A composition comprising lipase encoded by a lipase gene from Pseudomonas sp. ATCC 21808.

12. A method for hydrolysing an organic carboxylic ester comprising combining said ester with a lipase encoded by a lipase gene from Pseudomonas sp. ATCC 21808 in an aqueous medium, whereby said ester is hydrolysed.

13. A method for preparing lactones from hydroxycarboxylic acids, said method comprising combining said hydroxycarboxylic acid with a lipase encoded by a lipase gene from Pseudomonas sp. ATCC 21808 in an aqueous medium, whereby said lactone is formed.

14. A method for removing lipid stains from cloth, said method comprising;
    washing said cloth with a detergent formulation comprising a lipid removing amount of a lipase encoded by a lipase gene from Pseudomonas sp. ATCC 21808.

15. A detergent composition comprising a detergent-active composition and a lipase, wherein said lipase is produced by a Pseudomonas microorganism comprising a recombinant vector comprising a gene encoding said lipase and expressed in said Pseudomonas.

16. A detergent composition according to Claim 15, wherein said detergent composition is granular and comprising at least one of a builder, bleach or whitening agent.

17. A detergent composition according to Claim 15, wherein said detergent composition is liquid and comprising at least one of a bleach or whitening agent.

18. A detergent composition according to Claim 15, wherein said lipase is characterized by having the portrait amino acid sequence:

L-V-G-H-S-Q-G-G.

**19.** A liquid detergent composition comprising a lipase produced by expression of a gene encoding said lipase, said gene comprising a portion of a recombinant vector.

**20.** A granular detergent composition comprising a lipase produced by expression of a gene encoding said lipase, said gene comprising a portion of a recombinant vector.

**21.** A detergent composition comprising lipase encoded by a lipase gene from Pseudomonas sp. ATCC 21808.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 334 462 (GIST BROCADES N.V.) * Page 3, lines 26-36; page 8, lines 45-58; page 9, lines 1-9,14-35 * | 1,5,6,8 ,9,12- 14,21 | C 12 N 15/55 C 12 N 1/21 C 12 N 9/20 |
| Y | EP-A-0 218 272 (GIST BROCADES N.V.) * Page 8, column 1, lines 46-58; column 2, lines 36-47; page 9, column 2, lines 12-19; page 11, column 2, lines 35-41; claims 1-5,15-17,19 * | 1-5,12- 14,21 | C 12 P 7/62 C 12 P 17/02 C 11 D 3/386 // C 12 R 1/38 |
| Y | EP-A-0 331 376 (AMANO PHARMACEUTICAL CO., LTD) * Page 2, lines 13-40; page 5, lines 7-10; page 7, lines 18-19; claims 1,6,10 * | 1,5,6,8 ,9 | |
| Y | JOURNAL OF GENERAL MICROBIOLOGY, vol. 134, 1988, pages 433-440, SGM; S. WOHLFARTH et al.: "Chromosomal mapping and cloning of the lipase gene of Pseudomonas aeruginosa" * Page 435, "Results"; page 439 * | 1,8 | |

TECHNICAL FIELDS SEARCHED (Int. Cl.5)

C 12 N
C 11 D

The present search report has been drawn up for all claims

| Place of search | Date of completion of the search | Examiner |
|---|---|---|
| THE HAGUE | 02-11-1990 | NAUCHE S.A. |

EPO FORM 1503 03.82 (P0401)

European Patent
Office

---

## | CLAIMS INCURRING FEES

The present European patent application comprised at the time of filing more than ten claims.

☐ All claims fees have been paid within the prescribed time limit. The present European search report has been drawn up for all claims.

☐ Only part of the claims fees have been paid within the prescribed time limit. The present European search report has been drawn up for the first ten claims and for those claims for which claims fees have been paid,

namely claims:

☐ No claims fees have been paid within the prescribed time limit. The present European search report has been drawn up for the first ten claims.

---

## x | LACK OF UNITY OF INVENTION

The Search Division considers that the present European patent application does not comply with the requirement of unity of invention and relates to several inventions or groups of inventions,
namely:

### See sheet -B-

☐ All further search fees have been paid within the fixed time limit. The present European search report has been drawn up for all claims.

☐ Only part of the further search fees have been paid within the fixed time limit. The present European search report has been drawn up for those parts of the European patent application which relate to the inventions in respect of which search fees have been paid,

namely claims:

☒ None of the further search fees has been paid within the fixed time limit. The present European search report has been drawn up for those parts of the European patent application which relate to the invention first mentioned in the claims,

namely claims: 1-14,18,21

## LACK OF UNITY OF INVENTION

The Search Division considers that the present European patent application does not comply with the requirement of unity of invention and relates to several inventions or groups of inventions, namely:

1. Claims 1-14,18,21: Lipase, derived from the strain ARCC 21 808 (containing sequence L-V-G-H-S-Q-G-G), produced by Pseudomonas sp and uses of this lipase.

2. Claims 15-17: A detergent composition containing a lipase produced by a Pseudomonas.

3. Claims 19-20: A detergent, liquid or granular, comprising a lipase produced by a gene encoding said lipase, said gene comprising a portion of a recombinant vector.